# EUROPEAN PATENT APPLICATION

(11) **EP 2 011 472 A1**
(43) Date of publication of application: **07.01.2009**
(21) Application number: 07740331.9
(22) Date of filing: 29.03.2007
(51) Int. Cl.: A61K 8/11, A61K 8/14, A61K 8/31

(54) **CASEIN NANOPARTICLE**

(30) Priority: 29.03.2006 JP 2006090205
(71) Applicant: FUJIFILM Corporation, Minato-ku Tokyo 106-0031 (JP)
(72) Inventor: AIMI, Makiko, Minami-ashigara-shi, Kanagawa 250-0193 (JP); NEMORI, Ryoichi, Minami-ashigara-shi, Kanagawa 250-0193 (JP); OGIWARA, Kazutaka, Minami-ashigara-shi, Kanagawa 250-0193 (JP)
(74) Representative: HOFFMANN EITLE
(86) International application number: PCT/JP2007/056893
(87) International publication number: WO 2007/114262

(57) **Abstract**

An object of the present invention to provide: a nanoparticle, which can be produced without the use of surfactants and synthetic polymers, which has a controllable size and is stable in the acidic range, and which further contains an active substance; and a method for producing the same. The present invention provides a casein nanoparticle which contains an active substance and has an average particle size between 10 nm or more and less than 300 nm, which is produced by the following steps (a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5.

## Description

### TECHNICAL FIELD

The present invention relates to a nanoparticle for use in fields such as life science or medical diagnosis. More particularly, the present invention relates to a casein nanoparticle comprising an active substance.

### BACKGROUND ART

Fine particle materials have been expected to be widely used in biotechnology. Particularly, studies have been conducted actively in recent years on the application of nanoparticle materials developed by the advances of nanotechnology to biotechnology or medical care. Many study results have been reported.

Nanoparticles have been expected strongly from the early stage in the field of a drug delivery system (DDS) and are exceedingly promising as a carrier for drugs or genes. Particularly, studies using polymer micelle have been conducted actively. In most cases, AB- or ABA-type block copolymers are used because of the simplicity of their structures. The polymer micelle is characterized by its large drug capacity, high water solubility, high structural stability, non-accumulation, small particle size (100 nm or smaller), and functional separation. From this viewpoint, studies intended for targeting to target sites and solubilization of hydrophobic drugs have been conducted.

In recent years, cosmetics have embodied various novel techniques including nanotechnology and have thereby achieved improvement in functionality and usability and differentiation from other companies' products. More distinct effects on the skin have been demanded for cosmetics. Since the skin generally contains the stratum corneum as a barrier, the skin infiltration property of a drug is low. Improvement in the skin permeability of an active ingredient is essential for exerting sufficient effects on the skin. Many ingredients, even if having high effectiveness to the skin, are difficult to make into preparations because they have poor storage stability and are apt to cause skin irritation. The development of various capsules intended for improvement in percutaneous absorbability and storage stability, reduction in skin irritation, and so on have been pursued to solve these problems. Studies have been conducted currently on ultrafine emulsification or a variety of raw materials such as liposomes (e.g., Non-Patent Document I). However, surfactants used in emulsification raise safety concerns. Moreover, structure formation with ion complexes produces poor stability as compared with that with covalent bond.

The use of polymer materials is expected to considerably improve storage stability and in-vivo particle stability. However, most studies use synthetic polymers produced by emulsion polymerization or the like. Although toxicity is reduced in the synthetic polymers as compared with low-molecular substances, toxicity to some extent should be expected. Therefore, a safer carrier has been demanded.

Natural polymers exhibit high structural stability as with synthetic polymers and have safety much higher than that of synthetic polymers. Thus, the natural polymers have advantages as a DDS carrier. However, a difficult point of the natural polymer carrier as compared with synthetic polymers is a method for producing particles. Spray drying, freeze drying, and jet milling can be utilized as methods for producing natural polymer particles. However, in most cases, the particle size is a micron size and is difficult to control.

Patent Document 1 has proposed a solid preparation in the form of a multicore structure comprising active compounds. However, the multicore structure is 5 to 3000 µm in particle size. Moreover, Patent Document 2 has proposed a nanoparticle percutaneous absorbent using a polymer material. However, this absorbent is a product emulsified with a surfactant and raises safety and stability concerns as described above. Furthermore, Patent Document 3 has described a spherical protein particle. However, the spherical protein particle is 1 µm or larger in particle size as a composition comprising a drug. For previously known polymer nanoparticles including those described above, surfactants, polymerizable monomers, chemical crosslinking agents, and so on are employed in a particle formation process using even natural polymers, not to mention synthetic polymers. Thus, these polymer nanoparticles raise safety concerns.

Meanwhile, casein is a protein insoluble in water contained in milk. Since its hydrophobic portion is exposed, casein is apt to form aggregates. 10 to 100 caseins gather and form submicelle of approximately 20 nm. Furthermore, 100 to 1000 caseins gather and form casein micelle of 90 to 150 nm. The casein micelles further gather and form a micelle associate of approximately 500 nm. Thus, casein micelle has a wide size distribution and is aggregated when supplemented with a salt of sodium, potassium, or the like or placed at an acidic pH.
[Non-Patent Document 1] Mitsuhiro Nishida, Fragrance Journal, the Nov. issue, 17 (2005)
[Patent Document 1] Japanese Patent Laid-Open No. 2002-204673
[Patent Document 2] Japanese Patent Laid-Open No. 2002-308728
[Patent Document 3] National Publication of International Patent Application No. 2005-500304

### DISCLOSURE OF THE INVENTION

### Problems to be Solved by the Invention

It is an object of the present invention to solve the aforementioned problems of the prior art techniques. Specifically, it is an object of the present invention to provide: a nanoparticle, which can be produced without the use of surfactants and synthetic polymers, which has a controllable size and is stable in the acidic range, and which further contains an active substance; and a method for producing the same.

### Means for Solving the Problems

As a result of intensive studies directed towards achieving the aforementioned object, the present inventors have found that a nanoparticle containing an active substance can be produced by dissolving casein in a basic aqueous medium between pH 8 or more and less than pH 11, adding at least one type of active substance thereto, and injecting the resulting solution into an acidic aqueous medium at pH 3.5 to pH 7.5, or decreasing the pH of the solution to a pH value that is pH 1 or more away from the isoelectric point, thereby completing the present invention.

That is to say, the present invention provides a casein nanoparticle which contains an active substance and has an average particle size between 10 nm or more and less than 300 nm, which is produced by the following steps (a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5.

Another aspect of the present invention provides a casein nanoparticle which contains an active substance and has an average particle size between 10 nm or more and less than 50 nm, which is produced by the following steps (a) to (c):
(a) a step of mixing casein into a basic aqueous medium between pH 8 or more and less than pH 11;
(b) a step of adding at least one type of active substance to the solution obtained in the step (a); and
(c) a step of decreasing the pH of the solution obtained in the step (b) to a pH value that is pH 1 or more away from the isoelectric point, while stirring the solution.

Preferably, the active substance is an ionic substance or a fat-soluble substance.

Preferably, the active substance is a cosmetic ingredient, a functional food ingredient, or a pharmaceutical ingredient.

Preferred examples of such a cosmetic ingredient include a moisturizer, a skin-lightening agent, a hair growth stimulant, a hair restorer, a hair growing agent, an anti-white hair agent, an anti-aging agent, an antioxidant, a collagen synthesis promoter, an anti-wrinkle agent, an anti-acne agent, vitamin, an ultraviolet absorber, an aromatic, a coloring agent, an anhidrotic, a cooling agent, a warming agent, a melanin generation suppressant, a melanocyte activator, a cleansing agent, and a slimming agent. Preferred examples of such a functional food ingredient include vitamin, mineral, an antioxidant, an anti-stress agent, a nutritious supplement, amino acids, carotenoid, and fruit and vegetable extracts. Preferred examples of such a pharmaceutical ingredient include a hair growth stimulant, a hair restorer, a hair growing agent, an antibiotic, an anti-cancer agent, an anti-inflammatory agent, an antiallergic agent, a hormone agent, an antithrombotic agent, an immunosuppressive agent, a therapeutic agent for skin disease, an antifungal agent, a nucleic acid agent, an anesthetic, an antipyretic, an analgesic, an antipruritic agent, an antihydropic, an antitussive expectorant, an antiepileptic, an antiparkinson agent, a sedative hypnotic, an antianxiety agent, an analeptic, an agent for psychoneurosis, a muscle relaxant, an antidepressant, a combination cold remedy, an autonomic agent, a spasmolytic agent, a sweater, an anhidrotic, a cardiac stimulant, a therapeutic agent for arrhythmia, an antiarrhythmic agent, an angiotonic, a vasodilator, an antiarrhythmic agent, a hypotensive agent, an antidiabetic agent, a therapeutic agent for hyperlipidemia, a respiratory stimulant, an antitussive agent, vitamin, a remedy for parasitic skin disease, a homeostatic regulator, polypeptide, hormone, a parakeratosis suppressant, vaccine, and a skin softener.

Preferably, the casein nanoparticle of the present invention comprises 0.1% to 100% by weight of the active substance with respect to the weight of casein.

Preferably, a solution of the active substance dissolved in water or an organic solvent miscible at least at 10% by weight with water is added in the step (b).

Preferably, 0.1% to 100% by weight of the organic solvent miscible at least at 10% by weight with water is added with respect to the weight of the basic aqueous medium.

Preferably, an aqueous liposome dispersion containing the active substance is added in the step (b).

Preferably, the added liposome contains 0.1% to 100% by weight of the active substance with respect to the weight of casein.

Preferably, a cyclodextrin solution of the active substance is added in the step (b).

Preferably, 0.1% to 100% by weight of cyclodextrin is added with respect to the weight of casein.

Preferably, 0.1% to 100% by weight of lipid is added with respect to the weight of casein.

Preferably, 0.1 % to 100% by weight of a different type of protein is added with respect to the weight of casein.

Preferably, 0.1 % to 100% by weight of a cationic or anionic polysaccharide is added with respect to the weight of casein.

Preferably, 0.1 % to 100% by weight of a cationic or anionic protein is added with respect to the weight of casein.

Another aspect of the present invention provides a drug delivery agent which comprises the aforementioned casein nanoparticle of the present invention.

Preferably, the drug delivery agent is used as a transdermal agent, a local therapeutic agent, an oral therapeutic agent, a cosmetic product, a functional food, or a supplement.

Preferably, the amount of ethanol contained in the drug delivery agent is 20% or less.

Preferably, an additive is contained in the drug delivery agent.

Preferably, the additive is one or more types selected from among a moisturizer, a softener, a percutaneous absorption promoter, an antiseptic, a coloring agent, an aromatic, and a pH adjuster.

A further another aspect of the present invention provides a method for producing a casein nanoparticle containing an active substance and having an average particle size between 10 nm or more and less than 300 nm, which comprises the following steps (a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5.

A further another aspect of the present invention provides a method for producing a casein nanoparticle containing an active substance and having an average particle size between 10 nm or more and less than 50 nm, which comprises the following steps (a) to (c):
(a) a step of mixing casein into a basic aqueous medium between pH 8 or more and less than pH 11;
(b) a step of adding at least one type of active substance to the solution obtained in the step (a); and
(c) a step of decreasing the pH of the solution obtained in the step (b) to a pH value that is pH 1 or more away from the isoelectric point, while stirring the solution.

### BEST MODE FOR CARRYING OUT THE INVENTION

The embodiments of the present invention will be described in detail below.

The present invention relates to a casein nanoparticle containing an active substance and having an average particle size between 10 nm or more and less than 300 nm, which is produced by the following steps (a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5, or decreasing the pH of the solution obtained in the step (b) to a pH value that is pH 1 or more away from the isoelectric point, while stirring the solution.

In the present invention, it was found that a casein nanoparticle with a desired size can be produced. In addition, it was also found that an active substance can be incorporated into a casein nanoparticle by utilizing the interaction between an fat-soluble active substance and a hydrophobic portion of the casein. Moreover, it was also found that such casein nanoparticles are stably present in an aqueous solution.

As a fat-soluble substance, ClogP is preferably greater than 0, ClogP is more preferably 1 or greater, and ClogP is further preferably 3 or greater.

Furthermore, it was also found that the casein nanoparticle can incorporate an ionic active substance therein by use of a mixed particle of casein with an ionic polysaccharide or with a different type of ionic protein.

Specifically, according to the present invention, a nanoparticle containing a highly safe active substance can be produced without the use of surfactants and synthetic polymers.

The casein nanoparticle of the present invention has an average particle size usually between 10 nm or more and less than 300 nm, preferably between 10 and 100 nm, more preferably between 10 and 50 nm.

The casein nanoparticle of the present invention comprises at least one type of active substance. The amount of the active substance is not particularly limited. The casein nanoparticle generally comprises 0.1% to 100% by weight of the active substance with respect to the weight of casein.

The origin of the casein used in the present invention is not particularly limited, and it may be derived from milk or from beans. Examples of such casein that can be used in the present invention include α-casein, β-casein, γ-casein, κ-casein, and the mixtures thereof. A genetically modified product can also be used. Preferably, the casein of the present invention can be used in the form of casein sodium. These caseins can be used alone or in combination of two or more types.

The method for producing the casein nanoparticle of the present invention includes a method comprising mixing casein into a basic aqueous medium solution and injecting the resulting solution into an acidic aqueous medium and a method comprising mixing casein into a basic aqueous medium solution and decreasing the pH of the resulting solution, while stirring the solution.

Preferably, the method comprising mixing casein into a basic aqueous medium solution and injecting the resulting solution into an acidic aqueous medium is performed by use of a syringe because of the simplicity of its operation. However, the method is not particularly limited as long as it satisfies an injection rate, solubility, a temperature, and a stirring state. In general, the solution can be injected at an injection rate of 1 mL/min to 100 mL/min. The temperature of the basic aqueous medium can be set, as appropriate. It can be normally 0°C to 80°C, and preferably 25°C to 70°C. The temperature of the aqueous medium can be set, as appropriate. It can be normally 0°C to 80°C, and preferably 25°C to 60°C. A stirring speed can be set, as appropriate. It can be normally 100 rpm to 3 000 rpm, and preferably 200 rpm to 2000 rpm.

Preferably, the method comprising mixing casein into a basic aqueous medium solution and decreasing the pH of the resulting solution, while stirring the solution is performed by the dropping of an acid because of the simplicity of its operation. However, the method is not particularly limited as long as it satisfies solubility, a temperature, and a stirring state. The temperature of the basis aqueous medium can be set, as appropriate. It can be normally 0°C to 80°C, and preferably 25°C to 70°C. A stirring speed can be set, as appropriate. It can be normally 100 rpm to 3000 rpm, and preferably 200 rpm to 2000 rpm.

As the aqueous medium used in the present invention, an aqueous solution or buffer solution of an organic acid or base, or an aqueous solution or buffer solution of an inorganic acid or inorganic base can be used.

Specific examples thereof include, but are not limited to, aqueous solutions using organic acids such as citric acid, ascorbic acid, gluconic acid, carboxylic acid, tartaric acid, succinic acid, acetic acid, phthalic acid, trifluoroacetic acid, morpholinoethanesulfonic acid, and 2-[4-(2-hydroxyethyl)-1-piperazinyl]ethanesulfonic acid; organic bases such as tris(hydroxymethyl), aminomethane, and ammonia; inorganic acids such as hydrochloric acid, perchloric acid, and carbonic acid; and inorganic bases such as sodium phosphate, potassium phosphate, calcium hydroxide, sodium hydroxide, potassium hydroxide, and magnesium hydroxide.

The concentration of the aqueous medium used in the present invention is preferably approximately 10 mM to approximately 500 mM, and more preferably approximately 10 mM to approximately 200 mM.

The pH of the basic aqueous medium used in the present invention is preferably between pH 8 or more and less than pH 11, more preferably between pH 9 or more and less than pH 11, and further more preferably between pH 9.5 and pH 10.5. An excessively high pH might cause hydrolysis or pose handling risks. Therefore, the range described above is preferable.

In the present invention, a temperature at which casein is mixed into the basic aqueous medium at pH 8 or more is preferably 0°C to 80°C, more preferably 10°C to 60°C, and further more preferably 20°C to 40°C.

The pH of the acidic aqueous medium used in the present invention is preferably pH 3.5 to pH 7.5, more preferably pH 4 to pH 6, and further more preferably pH 5 to pH 6. In the case of pH 3 or less, there is a tendency where the particle size becomes large. In a method comprising mixing casein into a basic aqueous medium and decreasing the pH value while stirring the solution, the pH value obtained after the decrease of the pH is preferably between a pH value that is pH 1 or more away from the isoelectric point and pH 8.

The type of the active substance used in the present invention can be selected from among cosmetic ingredients and pharmaceutical ingredients, for example. Examples of such a cosmetic ingredient include a moisturizer, a skin-lightening agent, a hair growth stimulant, a hair restorer, a hair growing agent, an anti-white hair agent, an anti-aging agent, an antioxidant, a collagen synthesis promoter, an anti-wrinkle agent, an anti-acne agent, vitamin, an ultraviolet absorber, an aromatic, a coloring agent, an anhidrotic, a cooling agent, a warming agent, a melanin generation suppressant, a melanocyte activator, a cleansing agent, and a slimming agent. Examples of such a functional food ingredient include vitamin, mineral, an antioxidant, an anti-stress agent, a nutritious supplement, amino acids, carotenoid, and fruit and vegetable extracts. Examples of such a pharmaceutical ingredient include a hair growth stimulant, a hair restorer, a hair growing agent, an antibiotic, an anti-cancer agent, an anti-inflammatory agent, an antiallergic agent, a hormone agent, an antithrombotic agent, an immunosuppressive agent, a therapeutic agent for skin disease, an antifungal agent, a nucleic acid agent, an anesthetic, an antipyretic, an analgesic, an antipruritic agent, an antihydropic, an antitussive expectorant, an antiepileptic, an antiparkinson agent, a sedative hypnotic, an antianxiety agent, an analeptic, an agent for psychoneurosis, a muscle relaxant, an antidepressant, a combination cold remedy, an autonomic agent, a spasmolytic agent, a sweater, an anhidrotic, a cardiac stimulant, a therapeutic agent for arrhythmia, an antiarrhythmic agent, an angiotonic, a vasodilator, an antiarrhythmic agent, a hypotensive agent, an antidiabetic agent, a therapeutic agent for hyperlipidemia, a respiratory stimulant, an antitussive agent, vitamin, a remedy for parasitic skin disease, a homeostatic regulator, polypeptide, hormone, a parakeratosis suppressant, vaccine, and a skin softener. The aforementioned active substances can be used alone or in combination of two or more types.

Specific examples of the moisturizer used in the present invention are listed below. However, in the present invention, the moisturizer is not limited to these compounds. It includes hyaluronic acid, ceramide, Lipidure, isoflavone, amino acid, and collagen. The aforementioned moisturizers can be used alone or in combination of two or more types.

Specific examples of the skin-lightening agent used in the present invention are listed below. However, in the present invention, the skin-lightening agent is not limited to these compounds. It includes vitamin C derivatives, hydroquinones, arbutin, Rucinol, and ellagic acid. The aforementioned skin-lightening agents can be used alone or in combination of two or more types.

Specific examples of the anti-aging agent and the antioxidant used in the present invention are listed below. However, in the present invention, the anti-aging agent and the antioxidant are not limited to these compounds. They include carotenes, retinoic acid, retinol, vitamin C derivatives, kinetin, astaxanthin, tretinoin, vitamin E and a derivative thereof, sesamin, α-lipoic acid, coenzyme Q10, and flavonoids. The aforementioned anti-aging agents and antioxidants can be used alone or in combination of two or more types.

Specific examples of the anti-acne agent used in the present invention are listed below. However, in the present invention, the anti-acne agent is not limited to these compounds. It includes salicylic acid, resorcin, retinoic acid, nadifloxacin, an aminoglycoside antibiotic, a tetracycline antibiotic, and a lincomycin antibiotic. The aforementioned anti-acne agents can be used alone or in combination of two or more types.

Specific examples of an anticancer agent used in the present invention are listed below. However, in the present invention, the anticancer agent is not limited to these compounds. It includes, but are not limited to, fluorinated pyrimidine-based antimetabolites (e.g., 5-fluorouracil (5FU), tegafur, doxifluridine, and capecitabine); antibiotics (e.g., mitomycin (MMC) and Adriacin (DXR)); purine antimetabolites (e.g., folic acid antimetabolites (such as methotrexate) and mercaptopurine); active metabolites of vitamin A (e.g., antimetabolites (such as hydroxycarbamide), tretinoin, and tamibarotene); molecular target drugs (e.g., Herceptin and imatinib mesilate); platinum preparations (e.g., Briplatin or Randa (CDDP), Paraplatin (CBDC), Elplat (Oxa), and Aqupla); plant alkaloid drugs (e.g., Topotecin or Campto (CPT), taxol (PTX), Taxotere (DTX), and etoposide); alkylating agents (e.g., busulfan, cyclophosphamide, and ifomide); anti-androgen drugs (e.g., bicalutamide and flutamide); estrogen drugs (e.g., fosfestrol, chlormadinone acetate, and estramustine phosphate); LH-RH drugs (e.g., Leuplin and Zoladex); anti-estrogen drugs (e.g., tamoxifen citrate and toremifene citrate); aromatase inhibitors (e.g., fadrozole hydrochloride, anastrozole, and exemestane); corpus luteum hormone drugs (e.g., medroxyprogesterone acetate); and BCG. The aforementioned anticancer agents can be used alone or in combination of two or more types.

Specific examples of the antiallergic agent used in the present invention are listed below. However, in the present invention, the antiallergic agent is not limited to these compounds. It includes: mediator release inhibitors such as sodium cromoglycate and tranilast; histamine H1 antagonists such as ketotifen fumarate and azelastine hydrochloride; thromboxane inhibitors such as ozagrel hydrochloride; leukotriene antagonists such as pranlukast; and suplatast tosilate. The aforementioned antiallergic agents can be used alone or in combination of two or more types.

Specific examples of the immunosuppressive agent used in the present invention are listed below. However, in the present invention, the immunosuppressive agent is not limited to these compounds. It includes rapamycin, tacrolimus, cyclosporine, prednisolone, methylprednisolone, mycophenolate mofetil, azathioprine, and mizoribine. The aforementioned immunosuppressive agents can be used alone or in combination of two or more types.

The type of the hair growing ingredient used in the present invention is not particularly limited. Such hair growing ingredient can be selected from among cosmetic ingredients and pharmaceutical ingredients, for example. Specific examples of the hair growing ingredient contained in the protein nanoparticle of the present invention include: glycyrrhetic acid or a derivative thereof; glycyrrhizinic acid or a derivative thereof; hinokitiol; vitamin E or a derivative thereof; a vitamin C derivative; 6-benzylaminopurine; nicotinic acid amide; benzyl nicotinate; tocopherol nicotinate; nicotinic acid β-butoxy ester; isopropylmethylphenol; pentadecanoic acid or a derivative thereof; cepharanthin; finasteride; t-flavanone; an antioxidant such as carotenoid or kinetin; ethinyl estradiol; pantothenyl alcohol; pantothenyl ethyl ether; minoxidil or an analogue thereof; carpronium chloride; and adenosine. The aforementioned hair growing ingredients can be used alone or in combination of two or more types.

Specific examples of an organic solvent that is miscible at least at 10% by weight with water used in the present invention are listed below. However, in the present invention, the organic solvent is not limited to these compounds. Water-soluble organic solvents such as ethanol, isopropanol, ethylene glycol, glycerin, acetone, and THF are preferable.

In the present invention, the active substance can be added in the form of an aqueous liposome dispersion containing the active substance. Specific examples of lipid for forming the liposome used in the present invention are listed below. However, in the present invention, the lipid is not limited to these compounds. It includes egg-yolk lecithin, soybean lecithin, egg-yolk phosphatidylcholine, dipalmitoylphosphatidylcholine, and dimyristoylphosphatidylcholine. The liposome may also comprise phosphatidylserines, phosphatidylethanolamines, and cholesterol, in addition to the compounds described above.

Specific examples of cyclodextrin used in the present invention are listed below. However, in the present invention, the cyclodextrin is not limited to these compounds. It includes α-cyclodextrin, β-cyclodextrin, γ-cyclodextrin, 2,6-di-O-methyl-α-cyclodextrin, 2,6-di-O-methyl-β-cyclodextrin, glucuronyl glucosyl-β-cyclodextrin, heptakis(2,6-di-O-methyl)-β-cyclodextrin, 2-hydroxyethyl-β-cyclodextrin, hydroxypropyl-β-cyclodextrin, 6-O-α-maltosyl-α-cyclodextrin, methyl-β-cyclodextrin, 2,3,6-tri-O-methyl-β-cyclodextrin, and 6-O-α-D-glucosyl-α-cyclodextrin.

Specific examples of lipid used in the present invention are listed below. However, in the present invention, the lipid is not limited to these compounds. It includes phosphatidylcholine (lecithin), phosphatidylethanolamine, phosphatidylserine, phosphatidylinositol, phosphatidylglycerol, diphosphatidylglycerol, sphingosines, ceramide, oleic acid, linoleic acid, linolenic acid, palmitic acid, myristic acid, stearic acid, soybean oil, olive oil, and squalane.

A different type of protein used in the present invention is not particularly limited in kind. Preferably, a protein with a molecular weight of approximately 10,000 to 1,000,000 is used. The protein is not particularly limited in origin. Preferably, a protein derived from a human is used. Specific examples of the protein are listed below. However, in the present invention, the protein is not limited to these compounds. Examples of such a different type of protein that can be used herein include collagen, gelatin, albumin, transferrin, fibrin, fibrinogen, globulin, fibroin, laminin, fibronectin, and vitronectin. Moreover, the origin of the protein is not particularly limited, and any one of bovine, swine, fish and a genetically modified product can be used. Among them, gelatin and albumin are preferable.

An anionic polysaccharide used in the present invention is a polysaccharide having an acidic polar group such as a carboxyl group, a sulfuric acid group, or a phosphoric acid group. Specific examples thereof are listed below. However, in the present invention, the anionic polysaccharide is not limited to these compounds. It includes chondroitin sulfate, dextran sulfate, carboxymethyldextran, alginic acid, pectin, carrageenan, fucoidan, agaropectin, porphyran, karaya gum, gellan gum, xanthan gum, and hyaluronic acids.

A cationic polysaccharide used in the present invention is a polysaccharide having a basic polar group such as an amino group. Specific examples thereof are listed below. However, in the present invention, the cationic polysaccharide is not limited to these compounds. It includes polysaccharides comprising glucosamine (e.g., chitin and chitosan) or galactosamine as a constituent monosaccharide.

An anionic protein used in the present invention is a protein and a lipoprotein having an isoelectric point that is located on a more basic side than the physiological pH. Specific examples thereof are listed below. However, in the present invention, the anionic protein is not limited to these compounds. It includes polyglutamic acid, polyaspartic acid, cytochrome C, ribonuclease, trypsinogen, chymotrypsinogen, and α-chymotrypsin.

A cationic protein used in the present invention is a protein and a lipoprotein having an isoelectric point that is located on a more acidic side than the physiological pH. Specific examples thereof are listed below. However, in the present invention, the cationic protein is not limited to these compounds. It includes polylysine, polyarginine, histone, protamine, and ovalbumin.

Preferably, the ionic protein and polysaccharide used in the present invention have a charge opposite to the charge of the active substance. Preferably, the amount of the ionic protein or polysaccharide added is 0.1% to 100% by weight with respect to the weight of casein.

The casein nanoparticle of the present invention comprises the active substance therein. Such a casein nanoparticle comprising the active substance can be administered to the affected part for use. Specifically, the casein nanoparticle of the present invention is useful as a drug delivery agent.

In the present invention, the usage of the drug delivery agent is not particularly limited. For example, the drug delivery agent is used as a transdermal agent, a local therapeutic agent, an oral therapeutic agent, a cosmetic product, a supplement, and the like.

In the present invention, the drug delivery agent preferably comprises 0.01% to 50% by weight of the protein nanoparticle, and more preferably comprises 0.1 % to 10% by weight of the protein nanoparticle.

The amount of ethanol contained in the drug delivery agent is preferably 20% or less, and more preferably 10% or less.

In the present invention, the drug delivery agent may comprise an additive. The type of such additive is not particularly limited. Examples of such additive include a moisturizer, a softener, a percutaneous absorption promoter, an antiseptic, a coloring agent, an aromatic, and a pH adjuster.

Specific examples of the moisturizer that can be used in the present invention include, but are not limited to, agar, diglycerin, distearyldimonium hectorite, butylene glycol, polyethylene glycol, propylene glycol, sodium hyaluronate, hexylene glycol, coix seed extract, and vaserine.

Specific examples of the softener that can be used in the present invention include, but are not limited to, glycerin, mineral oil, and emollient ingredients (e.g. isopropyl isostearate, polyglyceryl isostearate, isotridecyl isononanoate, octyl isononanoate, oleic acid, glyceryl oleate, cacao butter, cholesterol, mixed fatty acid triglyceride, dioctyl succinate, sucrose acetate stearate, cyclopentanesiloxane, sucrose distearate, octyl palmitate, octyl hydroxystearate, arachidyl behenate, sucrose polybehenate, polymethylsilsesquioxane, myristyl alcohol, cetyl myristate, myristyl myristate, and hexyl laurate).

Specific examples of the percutaneous absorption promoter that can be used in the present invention include, but are not limited to, ethanol, isopropyl myristate, citric acid, squalane, oleic acid, menthol, N-methyl-2-pyrrolidone, diethyl adipate, diisopropyl adipate, diethyl sebacate, diisopropyl sebacate, isopropyl palmitate, isopropyl oleate, octyldodecyl oleate, isostearyl alcohol, 2-octyldodecanol, urea, vegetable oil, and animal oil.

Specific examples of the antiseptic that can be used in the present invention include, but are not limited to, benzoic acid, sodium benzoate, ethylparaben, potassium sorbate, sodium sorbate, sorbic acid, sodium dehydroacetate, and methylparaben.

Specific examples of the coloring agent that can be used in the present invention include, but are not limited to, kaoline, carmine, ultramarine blue, chromium oxide, and iron oxide.

Specific examples of the pH adjuster that can be used in the present invention include, but are not limited to, sodium citrate, sodium acetate, sodium hydroxide, potassium hydroxide, and phosphoric acid.

Preferred methods of administering the casein nanoparticle of the present invention include transdermal and transmucosal absorption. Specific examples of such administration method that can be applied in the present invention include, but are not limited to, an external liquid preparation, a poultice, an embrocation, a cleaning agent, a bath preparation, a disinfectant, an ointment, a gel, a cream, a paste, a cataplasm, a plaster, a wound surface-coating agent, a wound surface-coating gauze, a hemostatic, an adhesive, an adhesive tape, a percutaneous-absorption-type adhesive tape, a wound surface protecting agent, an aerosol, a lotion, a tonic, a liniment, an emulsion, a suspension, a saturant, a tincture, a powder, a foam, a cosmetic lotion, a massage cream, a nourishing cream, a pack, a sheet-form external skin preparation, a skin-adhesive-type cosmetic product, a lipstick, a makeup base, a foundation, a shampoo, a conditioner, a body soap, a soap, a bath form, a transnail agent, a nasal mucosal agent, an oral mucosal agent, a rectal mucosal agent, a vaginal mucosal agent, an eye mucosal agent, and a lung mucosal agent.

The dose of the casein nanoparticle of the present invention can be set appropriately according to the body weight of a patient, the state of the disease, and so on. In general, approximately 10 µg to 100 mg/µg can be administered per administration. Preferably, approximately 20 µg to 50 mg/kg can be administered per administration.

The present invention will be described more specifically in the following examples. However, these examples are not intended to limit the scope of the present invention.

### EXAMPLES

### Example 1

20 mg of casein (derived from milk; manufactured by Wako Pure Chemical Industries, Ltd.) and 2 mg of chondroitin sulfate-C (manufactured by Wako Pure Chemical Industries, Ltd.) were mixed into 1 mL of 50 mM phosphate buffer at pH 10. 5 mg of tocopherol (manufactured by Wako Pure Chemical Industries, Ltd.) was dissolved in 1 mL of ethanol. These two solutions were mixed and exposed to ultrasonic waves. Thereafter, 1 mL of the resulting mixture solution was injected into 10 mL of 200 mM phosphate buffer at pH 5, using a microsyringe under stirring conditions of 800 rpm and an external temperature of 40°C, so as to obtain casein nanoparticles.

The average particle size of the nanoparticles was measured with a light scattering photometer Nanotrac manufactured by Nikkiso Co., Ltd. As a result, the average particle size was found to be 57 nm.

### Example 2

1 mg of retinol (manufactured by Wako Pure Chemical Industries, Ltd.) and 20 mg of egg-yolk phosphatidylcholine (manufactured by NOF Corp.) were added to a 50-mL eggplant flask, and the mixture was then dissolved in 5 mL of ethanol. Thereafter, the ethanol was removed under reduced pressure using a rotary evaporator. 5 mL of 100 mM phosphate buffer at pH 9 was added to this solution, and the mixture was then shaken with a vortex mixer. The obtained suspension was irradiated with ultrasonic waves to obtain a liposome dispersion. 1 mL of the obtained liposome dispersion, 20 mg of casein (derived from milk; manufactured by Wako Pure Chemical Industries, Ltd.), and 1 mL of 200 mM phosphate buffer at pH 10 were mixed. 1 mL of the resulting solution was injected into 10 mL of phosphate buffer at pH 5, using a microsyringe under stirring conditions of 800 rpm and an external temperature of 40°C, so as to obtain casein nanoparticles.

The average particle size of the nanoparticles was measured with a light scattering photometer Nanotrac manufactured by Nikkiso Co., Ltd. As a result, the average particle size was found to be 60 nm.

### Example 3

5 mg of β-carotene (manufactured by Wako Pure Chemical Industries, Ltd.), 0.5 mL of ethanol, 20 mg of casein (derived from milk; manufactured by Wako Pure Chemical Industries, Ltd.), 5 mg of cyclodextrin (manufactured by Wako Pure Chemical Industries, Ltd.), and 1 mL of 50 mM phosphate buffer at pH 10 were mixed, and the mixture was then exposed to ultrasonic waves. 1 mL of the resulting solution was injected into 10 mL of 300 mM phosphate buffer at pH 5, using a microsyringe under stirring conditions of 800 rpm and an external temperature of 40°C, so as to obtain casein nanoparticles.

The average particle size of the nanoparticles was measured with a light scattering photometer Nanotrac manufactured by Nikkiso Co., Ltd. As a result, the average particle size was found to be 88 nm.

### Example 4

20 mg of casein (derived from milk; manufactured by Wako Pure Chemical Industries, Ltd.) and 1 mg of protamine sulfate (manufactured by Wako Pure Chemical Industries, Ltd.) were mixed into 1 mL of 50 mM phosphate buffer at pH 10. 1 mg of α-lipoic acid was dissolved in ion-exchanged water. These two solutions were mixed. 1 mL of the resulting mixture solution was injected into 10 mL of 200 mM phosphate buffer at pH 5, using a microsyringe under stirring conditions of 800 rpm and an external temperature of 40°C, so as to obtain casein nanoparticles.

The average particle size of the nanoparticles was measured with a light scattering photometer Nanotrac manufactured by Nikkiso Co., Ltd. As a result, the average particle size was found to be 48 nm.
* When the particle size measurement device was changed with another device, a small value was obtained as a average particle size. The average particle size on an electron micrograph was close to this value.

### Example 5

100 mg of casein sodium (derived from milk; manufactured by Wako Pure Chemical Industries, Ltd.) was mixed into 10 mL of 100 mM borate buffer at pH 10. 1 mg of tocopherol acetate was dissolved in 0.1 mL of ethanol. Thereafter, 5 mg of glycyrrhetic acid was dissolved in 1 mL of ethanol. These two types of solutions were mixed. While stirring, hydrochloric acid was added to the mixed solution to adjust the pH of the solution to pH 7, so as to obtain casein nanoparticles.

The average particle size of the nanoparticles was measured with a light scattering photometer Nanotrac manufactured by Nikkiso Co., Ltd. As a result, the average particle size was found to be 32 nm.

### Example 6

100 mg of casein (derived from milk; manufactured by Wako Pure Chemical Industries, Ltd.) was mixed into 10 mL of 50 mM phosphate buffer at pH 10. 3.4 mg of glycyrrhetic acid (manufactured by Wako Pure Chemical Industries, Ltd.) was dissolved in 0.1 mL of ethanol. These two types of solutions were mixed. While stirring, hydrochloric acid was added to the mixed solution to adjust the pH of the solution to pH 7, so as to obtain casein nanoparticles.

The average particle size of the nanoparticles was measured with a light scattering photometer Nanotrac manufactured by Nikkiso Co., Ltd. As a result, the average particle size was found to be 23 nm.

### Example 7

200 mg of casein sodium (derived from milk; manufactured by Wako Pure Chemical Industries, Ltd.) was mixed into 10 mL of 200 mM phosphate buffer at pH 10. 8.5 mg of benzyl nicotinate was dissolved in 0.15 mL of ethanol. These two types of solutions were mixed. While stirring, hydrochloric acid was added to the mixed solution to adjust the pH of the solution to pH 7, so as to obtain casein nanoparticles.

The average particle size of the nanoparticles was measured with a light scattering photometer Nanotrac manufactured by Nikkiso Co., Ltd. As a result, the average particle size was found to be 27 nm.

### Example 8

50 mg of casein sodium (derived from milk; manufactured by Wako Pure Chemical Industries, Ltd.) was mixed into 10 mL of 25 mM phosphate buffer at pH 10. 1.7 mg of hinokitiol was dissolved in 0.1 mL of ethanol. These two types of solutions were mixed. While stirring, hydrochloric acid was added to the mixed solution to adjust the pH of the solution to pH 7, so as to obtain casein nanoparticles.

The average particle size of the nanoparticles was measured with a light scattering photometer Nanotrac manufactured by Nikkiso Co., Ltd. As a result, the average particle size was found to be 16 nm.

### Comparative example 1

100 mg of casein sodium (derived from milk; manufactured by Wako Pure Chemical Industries, Ltd.) was mixed into 10 mL of distilled water. Thereafter, 3.4 mg of glycyrrhetic acid (manufactured by Wako Pure Chemical Industries, Ltd.) was dissolved in 0.1 mL of ethanol. These two types of solutions were mixed.

The average particle size of the nanoparticles was measured with a light scattering photometer Nanotrac manufactured by Nikkiso Co., Ltd. As a result, the average particle size was found to be 327 nm.

### Comparative example 2

100 mg of casein (derived from milk; manufactured by Wako Pure Chemical Industries, Ltd.) was mixed into 10 mL of 50 mM phosphate buffer at pH 10. 3.4 mg of glycyrrhetic acid (manufactured by Wako Pure Chemical Industries, Ltd.) was dissolved in 0.1 mL of ethanol. These two types of solutions were fixed. While stirring, hydrochloric acid was added to the mixed solution to adjust the pH of the solution to pH 4. As a result, the mixed solution became clouded and agglutinated.

### Comparative example 3

100 mg of casein sodium (derived from milk; manufactured by Wako Pure Chemical Industries, Ltd.) was mixed into 10 mL of 50 mM phosphate buffer at pH 12. 3.4 mg of glycyrrhetic acid (manufactured by Wako Pure Chemical Industries, Ltd.) was dissolved in 0.1 mL of ethanol. These two types of solutions were mixed. While stirring, hydrochloric acid was added to the mixed solution to adjust the pH of the solution to pH 7.

The average particle size of the nanoparticles was measured with a light scattering photometer Nanotrac manufactured by Nikkiso Co., Ltd. As a result, the average particle size was found to be 485 nm.

### Test example 1

The nanoparticles of Examples 5 to 8 were centrifuged with an ultracentrifuge CS100GXL manufactured by Hitachi Koki Co., Ltd. Based on the results of the HPLC analysis of the supernatant and the additive amount, the incorporation rate of an active substance contained in the particle was obtained.

**Table 1**

| | Active substance | ClogP value | Incorporation rate |
|---|---|---|---|
| Example 5 | Tocopherol acetate | 12.2 | 99% |
| Example 6 | glycyrrhetic acid | 6.3 | 99% |
| Example 7 | Benzyl nicotinate | 2.6 | 45% |
| Example 8 | hinokitiol | 1.9 | 38% |

As the casein nanoparticle of the present invention, a biocompatible casein is used without the use of surfactants and synthetic polymers. Thus, the present casein nanoparticle is highly safe. In addition, the casein nanoparticle of the present invention has a controllable size, and it is stable in the acidic range. Thus, in the present casein nanoparticle, an absorption rate and usability can be improved. Moreover, since the casein nanoparticle of the present invention contains an active substance, such active substance that is insoluble or only partially soluble in water can be stably dispersed in water.

## Claims

1. A casein nanoparticle which contains an active substance and has an average particle size between 10 nm or more and less than 300 nm, which is produced by the following steps
(a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5.

2. A casein nanoparticle which contains an active substance and has an average particle size between 10 nm or more and less than 50 nm, which is produced by the following steps (a) to (c):
(a) a step of mixing casein into a basic aqueous medium between pH 8 or more and less than pH 11;
(b) a step of adding at least one type of active substance to the solution obtained in the step (a); and
(c) a step of decreasing the pH of the solution obtained in the step (b) to a pH value that is pH 1 or more away from the isoelectric point, while stirring the solution.

3. The casein nanoparticle of claim 1 or 2, wherein the active substance is an ionic substance or a fat-soluble substance.

4. The casein nanoparticle of any of claims 1 to 3, wherein the active substance is a cosmetic ingredient, a functional food ingredient, or a pharmaceutical ingredient.

5. The casein nanoparticle of claim 4, wherein the active substance is a moisturizer, a skin-lightening agent, a hair growth stimulant, a hair restorer, a hair growing agent, an anti-white hair agent, an anti-aging agent, an antioxidant, a collagen synthesis promoter, an anti-wrinkle agent, an anti-acne agent, vitamin, an ultraviolet absorber, an aromatic, a coloring agent, an anhidrotic, a cooling agent, a warming agent, a melanin generation suppressant, a melanocyte activator, a cleansing agent, and a slimming agent; and the functional food ingredient is vitamin, mineral, an antioxidant, an anti-stress agent, a nutritious supplement, amino acids, carotenoid, and fruit and vegetable extracts; and the pharmaceutical ingredient is a hair growth stimulant, a hair restorer, a hair growing agent, an antibiotic, an anti-cancer agent, an anti-inflammatory agent, an antiallergic agent, a hormone agent, an antithrombotic agent, an immunosuppressive agent, a therapeutic agent for skin disease, an antifungal agent, a nucleic acid agent, an anesthetic, an antipyretic, an analgesic, an antipruritic agent, an antihydropic, an antitussive expectorant, an antiepileptic, an antiparkinson agent, a sedative hypnotic, an antianxiety agent, an analeptic, an agent for psychoneurosis, a muscle relaxant, an antidepressant, a combination cold remedy, an autonomic agent, a spasmolytic agent, a sweater, an anhidrotic, a cardiac stimulant, a therapeutic agent for arrhythmia, an antiarrhythmic agent, an angiotonic, a vasodilator, an antiarrhythmic agent, a hypotensive agent, an antidiabetic agent, a therapeutic agent for hyperlipidemia, a respiratory stimulant, an antitussive agent, vitamin, a remedy for parasitic skin disease, a homeostatic regulator, polypeptide, hormone, a parakeratosis suppressant, vaccine, and a skin softener.

6. The casein nanoparticle of any of claims 1 to 5, which comprises 0.1% to 100% by weight of the active substance with respect to the weight of casein.

7. The casein nanoparticle of any of claims 1 to 6, wherein a solution of the active substance dissolved in water or an organic solvent miscible at least at 10% by weight with water is added in the step (b).

8. The casein nanoparticle of claim 7, wherein 0.1% to 100% by weight of the organic solvent miscible at least at 10% by weight with water is added with respect to the weight of the basic aqueous medium.

9. The casein nanoparticle of any of claims 1 to 8, wherein an aqueous liposome dispersion containing the active substance is added in the step (b).

10. The casein nanoparticle of claim 9, wherein a liposome containing 0.1% to 100% by weight of the active substance with respect to the weight of casein is added.

11. The casein nanoparticle of any of claims 1 to 8, wherein a cyclodextrin solution of the active substance is added in the step (b).

12. The casein nanoparticle of claim 11, wherein 0.1% to 100% by weight of cyclodextrin is added with respect to the weight of casein.

13. The casein nanoparticle of any of claims 1 to 12, wherein 0.1% to 100% by weight of lipid is added with respect to the weight of casein.

14. The casein nanoparticle of any of claims 1 to 13, wherein 0.1 % to 100% by weight of a different type of protein is added with respect to the weight of casein.

15. The casein nanoparticle of any of claims 1 to 14, wherein 0.1% to 100% by weight of a cationic or anionic polysaccharide is added with respect to the weight of casein.

16. The casein nanoparticle of any of claims 1 to 15, wherein 0.1% to 100% by weight of a cationic or anionic protein is added with respect to the weight of casein.

17. A drug delivery agent which comprises the casein nanoparticle of any of claims 1 to 16.

18. The drug delivery agent of claim 17 which is used as a transdermal agent, a local therapeutic agent, an oral therapeutic agent, or a supplement.

19. The drug delivery agent of claim 17 or 18, wherein the amount of ethanol contained is 20% or less.

20. The drug delivery agent of any of claims 17 to 19, wherein an additive is contained in the drug delivery agent.

21. The drug delivery agent of claim 20, wherein the additive is one or more types selected from among a moisturizer, a softener, a percutaneous absorption promoter, an antiseptic, a coloring agent, an aromatic, and a pH adjuster.

22. A method for producing a casein nanoparticle containing an active substance and having an average particle size between 10 nm or more and less than 3 00 nm, which comprises the following steps (a) to (c):
(a) a step of mixing casein into a basic aqueous medium between pH 8 or more and less than pH 11;
(b) a step of adding at least one type of active substance to the solution obtained in the step (a); and
(c) a step of injecting the solution obtained in the step (b) into an acidic aqueous medium at pH 3.5 to pH 7.5.

23. A method for producing a casein nanoparticle containing an active substance and having an average particle size between 10 nm or more and less than 50 nm, which comprises the following steps (a) to (c):
(a) a step of mixing casein into a basic aqueous medium between pH 8 or more and less than pH 11;
(b) a step of adding at least one type of active substance to the solution obtained in the step (a); and
(c) a step of decreasing the pH of the solution obtained in the step (b) to a pH 3.5-7.5, while stirring the solution.
